# EUROPEAN PATENT SPECIFICATION

(11) **EP 2 489 661 B1**
(45) Date of publication and mention of the grant of the patent: **13.11.2013**
(21) Application number: 12163219.4
(22) Date of filing: 04.04.2012
(51) Int. Cl.: C07D 239/94, C07D 405/04, A61K 31/517, A61P 35/00

(54) **Impurity of lapatinib and salts thereof**
Unreinheit von Lapatinib und Salze davon
Impureté de lapatinib et sels correspondants

(30) Priority: 20.05.2011 IT MI20110894
(43) Date of publication of application: 22.08.2012
(73) Proprietor: F.I.S.- Fabbrica Italiana Sintetici S.p.A., 36075 Alte di Montecchio Maggiore (VI) (IT)
(72) Inventor: Fontana, Francesco, I-22030 Longone al Segrino (Como) (IT); Leganza, Alessandro, I-36040 Torri di Quartesolo (Vicenza) (IT); Osti, Sergio, I-35125 Padova (IT)
(74) Representative: Long, Giorgio

(56) References cited:
- WO-A1-99/35146
- WO-A2-2010/017387
- US-A1- 2003 220 354

## Description

### Technical field of the invention

An impurity of the active ingredient Lapatinib, methods for identifying it, quantifying it and for preventing or limiting the presence thereof in Lapatinib and salts thereof represents the object of the present invention.

### State of the art

Lapatinib is a pharmaceutical active ingredient used for the treatment of advanced metastatic lung cancer and it is currently available in the market under the name Tykerb® sold by GlaxoSmithKline (GSK).

According to the indications of the manufacturer, Tykerb® contains Lapatinib as a monohydrate ditosylate salt of formula (XIII-bis): 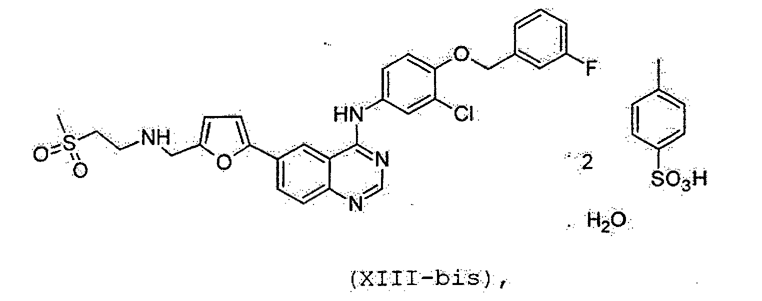 having the chemical name of N-{3-chloro-4-[(3-fluorobenzyl)oxy]phenyl}-6-[5-({[2-(methylsulfonyl)ethyl] amino}methyl)furan-2-yl]quinazoline-4-amine bis(4-methylbenzenesulfonate) monohydrate, CAS RN 388082-78-8 and melting point 250-256°C.

This substance can be prepared following the teachings of the prior art such as for example those contained in. US 7,157,466. In particular, in such reference, examples 10 and 11 show the preparation of monohydrate ditosylate salt starting from anhydrous ditosylate salt.

Just like any compound obtained by means of chemical synthesis, Lapatinib, or salts thereof, may contain small amounts of foreign compounds referred to as impurities. These impurities may be the raw materials, the synthetic intermediaries, reaction by-products, product degradation products etc. The impurities of Lapatinib just like those of any other pharmaceutical active ingredient or relative drug, referred to as "pharmaceutical impurities", may affect both the efficiency and the safety of a drug which, in extreme cases, could even be harmful for the patient. The purity of an active ingredient like the Lapatinib produced through a production process based on subsequent chemical reactions represents a critical factor as regards commercialization. The US Food and Drug Administration (FDA) and the European Medicinal Agency (EMA) as well as the relative pharmacopoeia require that the impurities be maintained below given limit values.

The product of a chemical reaction is rarely a single compound having purity sufficient to meet the regulatory standards. By-products due to secondary reactions of the reagents used in the reaction can also be present in the isolated product. In some steps of the production process of an active ingredient, such as Lapatinib, the purity is analysed, generally by means of high performance liquid chromatography (HPLC), gas chromatography (GC) or thin layer chromatography (TLC), for defining if it is suitable for the subsequent treatment and lastly for use in the pharmaceutical product.

Generally, the impurities are identified spectroscopically, thus a chromatographic peak position, such as that of a chromatogram or a spot on a TLC panel, is associated thereto.

Once a peak position has been associated to a particular impurity, the impurity can be identified is a sample for the relative position thereof in the chromatogram, where the position in the chromatogram is measured in minutes between the injection of the sample in a column and elution of the impurity through the detector. The position in the chromatogram is known as the retention time and the ratio between the retention times is known as the relative retention time. A man skilled in pharmaceutical art knows that a relatively pure compound may be used as a reference standard. A reference standard is similar to a reference marker, except for the fact that the latter can be used not only for detecting the impurities, but also for quantifying the amount of impurities present in the sample of active ingredient.

As known to those skilled in the art, the management of process impurities is considerably improved by understanding the chemical structures thereof, the synthetic process and identifying the parameters that affect the amount of impurities in the final product for example by means of DOE. The impurities of Lapatinib, including the intermediaries not entirely reacted, the impurities of the raw materials, the reaction by-products, the degradation products, as well as other products, may affect the quality and efficiency of the pharmaceutical form containing Lapatinib. Thus, there arises the need for a method for defining the level of impurities in samples of Lapatinib and methods for removing the impurities or limiting the content thereof or preventing the formation thereof.

### Summary of the invention

The present invention regards N-{3-chloro-4-[(2-fluorobenzyl)oxy]phenyl}-6-[5-({[2-(methylsulfonyl)ethyl]amino}methyl)furan-2-yl] quinazoline-4-amine compound of formula (I) or a salt thereof: 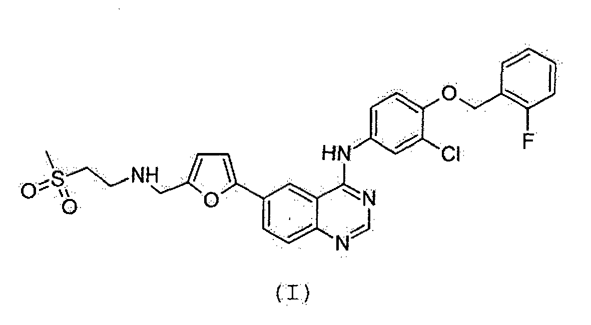 the analytical methods for detecting and quantifying this impurity in Lapatinib and salts thereof, the method of preparation and use thereof. The present invention also regards the method of synthesis of Lapatinib comprising less than 0.05 percent area or % weight on weight of the compound of formula (I) and also described is Lapatinib itself with such content of this impurity.

### Brief description of the figures (or drawings):

By way of example:
Figure 1 shows the 1H-NMR spectrum of Lapatinib monohydrate; ditosylate obtained according to the method of the present invention;
Figure 2 shows the 1H-NMR spectrum of the N-{3-chloro-4-[(2-fluorobenzyl)oxy]phenyl}-6-[5-({[2-(methylsulfonyl)ethy]amino}ethyl)furan-2-yl] quinazoline-4-amine compound of formula (I) as a ditosylate salt;
Figure 3 shows the 1H-NMR spectrum of the N-{3-chloro-4-[(2-fluorobenzyl)oxy]phenyl}-6-[5-({[2-(methylsulfonyl)ethyl]amino}methyl)furan-2-yl] quinazoline-4-amine compound of formula (I), as a free base;
Figure 4 shows the 1H-NMR spectrum of 5-[4-({3-chloro-4-[(2-fluorobenzyl)oxy]phenyl}amino)quinazoline-6-yl]furan-2-carbaldehyde of formula (II);
Figure 5 shows the 1H-NMR spectrum of N-{3-chloro-4-[(2-fluorobenzyl)oxy]phenyl}-6-iodoquinazoline-4-amine hydrochloride of formula (III);
Figure 6 shows the 1H-NMR spectrum of 3-chloro-4-[(2-fluorobenzyl)oxy]aniline of formula (IV);
Figure 7 shows the HPLC chromatogram of 3-chloro-4-[(3-fluorobenzyl)oxy]aniline of formula (X) containing 0.066 percent area (HPLC) of 3-chloro-4-[(2-fluorobenzyl)oxy]aniline impurity of formula (IV) used for preparing the Lapatinib monohydrate ditosylate of Figure 8;
Figure 8 shows the Lapatinib monohydrate ditosylate of formula (XIII - bis) containing 0.024 percent area (HPLC) of impurity of formula (I -bis) obtained according to the method of the invention.

### Detailed description of the invention

The present invention regards N-{3-chloro-4-[(2-fluorobenzyl)oxy]phenyl}-6-[5-({[2-(methylsulfonyl)ethyl]amino}methyl)furan-2-yl] quinazoline-4-amine compound of formula (I) or a salt thereof: 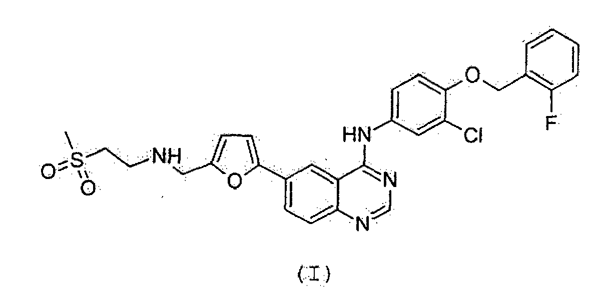 preferably in form of a ditosylate salt or as a monohydrate ditosylate salt of formula (I - bis): 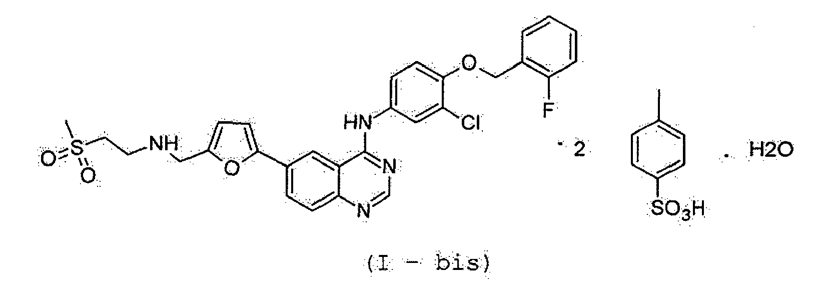 possibly also comprising 0.01 to 5.0 percent area (HPLC) of Lapatinib or a salt thereof or compound of formula (I) or a salt thereof having at least a 95.0 HPLC purity (area percent).

It was experimentally discovered that the compound of formula (I) is both an impurity of Lapatinib and a precursor of a genotoxic impurity of Lapatinib.

Actually, there was surprisingly observed the following degradation/hydrolysis mechanism: 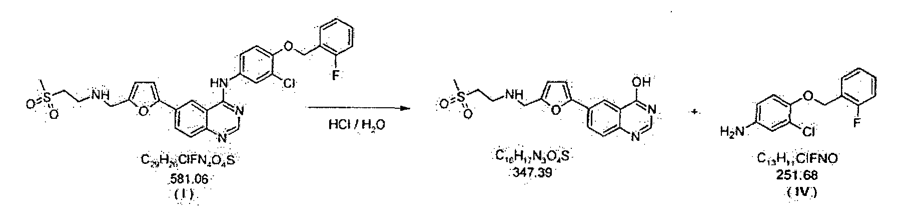 which leads to the formation of small but considerable amounts of 3-chloro-4-[(2-fluorobenzyl)oxy]aniline of formula (IV): 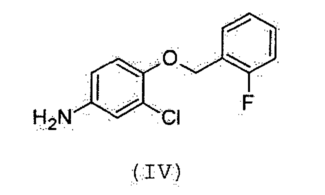 a substance which, as the aniline parent, is genotoxic. As a confirmation of the mutagenicity, the analogous with the fluorine in meta position instead of ortho position is classified as a Mutagen of category 3, with R68 risk phrase, in the ESIS database of the European Commission.

Thus, it is clear that it is essential to reduce as much as possible the amount of compound of formula (I), impurity of Lapatinib and precursor of genotoxic impurity of Lapatinib, in Lapatinib or salts thereof. For such purpose, Lapatinib or salts thereof comprising less than 0.05 percent area (HPLC) of this impurity guarantee the quality of the relative drug.

The impurity in question, the aforementioned degradation mechanism, as well as the relative genotoxic impurity, have never been described before according to our knowledge. Obviously, the removal of this specific impurity of Lapatinib or salts thereof has ever been considered as desirable as far as we know either.

The fact that the compound of formula (I) is an impurity of Lapatinib was not easy to define due to the fact that, during the development of the process of synthesis of Lapatinib monohydrate ditosylate neither these impurity nor the synthetic precursors thereof were ever observed due to the fact that, given the structural similarity with the active ingredient, through the HPLC methods used, it eluted together with the active ingredient and thus the signal thereof was covered by that of Lapatinib. Thus, there was no analytical evidence leading to detecting the presence thereof.

Furthermore, it was experimentally observed that this impurity of Lapatinib and all the synthetic precursors thereof behave, in terms of solubility and reactivity, exactly like Lapatinib and the synthetic precursors thereof and thus the impurity in the 3-chloro-4-[(2-fluorobenzyl)oxy]aniline of formula (IV) raw material: 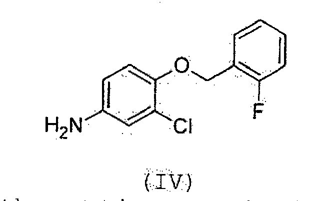 is kept during the entire synthesis up to Lapatinib monohydrate ditosylate.

The problem addressed by the present invention is thus that of providing Lapatinib or a salt thereof comprising an amount of impurity of formula (I) or a salt thereof lower than about 0.05 percent area by means of HPLC chromatographic analysis or lower than about 0.05 percent weight on weight. Preferably, Lapatinib is in form of Lapatinib monohydrate ditosylate.

This problem is resolved by means of the method of preparation of Lapatinib having less than 0.05 percent area (HPLC) or % weight on weight of this impurity, as well as by providing the compound of formula (I) or salts thereof, methods of identification and quantification in Lapatinib or salts thereof, as outlined in the attached claims, whose definitions form an integral part of the present description.

In order to reduce the amount of an impurity in an active ingredient it is necessary to detect the presence thereof using appropriate analytical methods, it is convenient to identify it, quantify it and only afterwards one can provide a method of synthesis capable of preventing the formation and/or provide for the removal thereof. However, this essentially requires providing the reference standard or reference marker of this impurity. For such purpose the compound of formula (I) can be conveniently prepared by means of a method comprising the following steps:
A) reacting 3-chloro-4-[(2-fluorobenzyl)oxy]aniline of formula (IV): 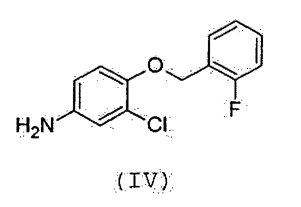 with 4-chloro-6-iodoquinazoline of formula (VIII): 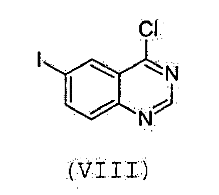 to yield N-{3-chloro-4-[(2-fluorobenzyl)oxy]phenyl}-6-iodoquinazoline-4-amine hydrochloride of formula (III): 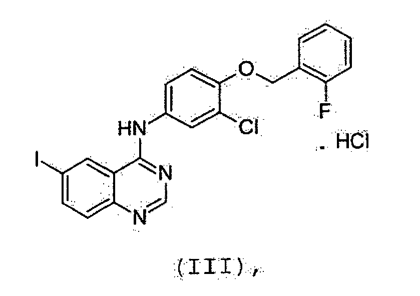
B) reacting the compound of formula (III) with the 2-formyl furan-5-boronic acid of formula (IX): 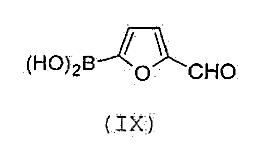 to yield 5-[4-({3-chloro-4-[(2-fluorobenzyl)oxy]phenyl}amino)quinazoline-6-yl]furan-2-carbaldehyde of formula (II): 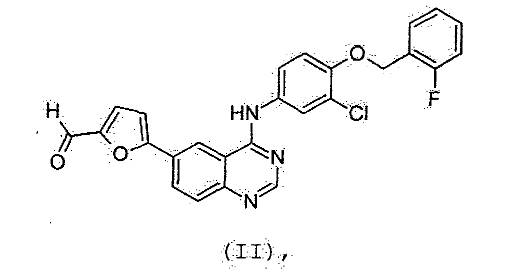
C) reacting the compound of formula (II) with 2-(methylsulfonyl)ethanamine hydrochloride to yield the compound of formula (I);
D) optionally the compound of formula (I) is converted into a ditosylate salt or a monohydrate ditosylate salt.

In particular, it is preferable that in step (b) the filtration of the inorganic salts present in the reaction mixture be carried out at a temperature comprised between 50°C and 60°C given that if performed at lower temperatures it can lead to considerable yield losses.

The N-{3-chloro-4-[(2-fluorobenzyl)oxy]phenyl}-6-iodoquinazoline-4-amine hydrochloride compound of formula (III): 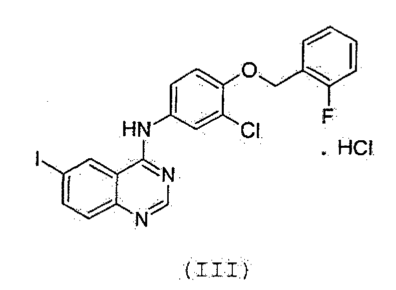 may also be obtained in a free base form. The 5-[4-({3-chloro-4-[(2-fluorobenzyl)oxy]phenyl}amino)quinazoline-6-yl]furan-2-carbaldehyde compound of formula (II): 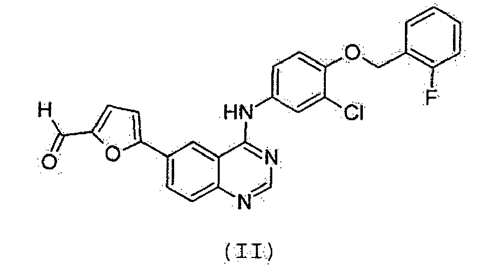 vice versa, may also be obtained in form of a salt, for example hydrochloride.

Once prepared, the compound of formula (I) according to the method of the invention above can also be conveniently used in a method for the identification of the compound of formula (I) in Lapatinib or a salt thereof comprising:
a) adding a known amount of compound of formula (I) or a salt thereof to the Lapatinib sample or a salt thereof,
b) carrying out HPLC analysis of the Lapatinib sample or a salt thereof of step a),
c) detecting the HPLC peak of the compound of formula (I) ;
or by means of the following method:
a1) analysing the compound of formula (I) or a salt thereof by means of HPLC,
b1) analysing the Lapatinib sample or a salt thereof by means of HPLC,
c1) detecting the HPLC peak of the compound of formula (I) by comparing the retention times or relative retention times.

Substantially using the method above may allow identifying the peak in the chromatogram of Lapatinib or a salt thereof regarding the impurity compound of formula (I). The analysis may be of the HPLC and GC type.

Besides the identification of the impurity peak in Lapatinib or a salt thereof regarding the compound of formula (I), it can also be quantified by means of the method comprising:
i) measuring the peak area corresponding to the compound of formula (I) in a Lapatinib sample or a salt thereof having an unknown amount of this compound by means of HPLC;
ii) measuring the peak area corresponding to a reference standard containing a known amount of compound of formula (I) or a salt thereof by means of HPLC,
iii) defining the amount of compound of formula (I) in Lapatinib or a salt thereof by comparing the area measured in the step a) with that measured in the step b).

It is thus clear that the compound of formula (I) or a salt thereof may be used as a reference marker or reference standard respectively for the identification and/or the quantification of the same in Lapatinib or a salt thereof.

Having these methods of identification and quantification allowed providing a method for preparing Lapatinib or a salt thereof, preferably monohydrate ditosylate, comprising an amount of impurity of formula (I) or a salt thereof lower than about 0.05 percent area by means of chromatographic analysis or lower than about 0.05 percent weight on weight, comprising the following steps:
1) providing 3-chloro-4-[(3-fluorobenzyl)oxy]aniline of formula (X): 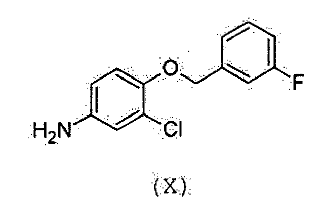 characterised in that it comprises less than about 0.15 percent area by means of chromatographic analysis or lower than about 0.15 percent weight on weight of 3-chloro-4-[(2-fluorobenzyl)oxy]aniline of formula (IV): 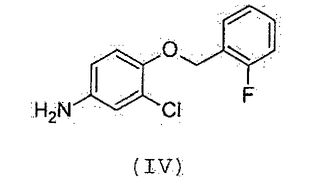
2) reacting 3-chloro-4-[(3-fluorobenzyl)oxy]aniline of formula (X) with 4-chloro-6-iodoquinazoline of formula (VIII): 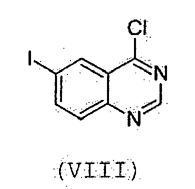 to yield N-{3-chloro-4-[(3-fluorobenzyl)oxy]phenyl}-6-iodoquinazoline-4-amine hydrochloride of formula (XI): 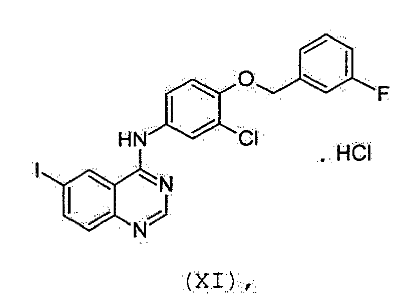
3) reacting the compound of formula (XI) with the 2-formyl furan-5-boronic acid of formula (IX): 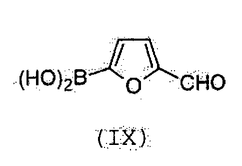 to yield 5-[4-({3-chloro-4-[(3-fluorobenzyl)oxy]phenyl}amino)quinazoline-6-yl]furan-2-carbaldehyde of formula (XII): 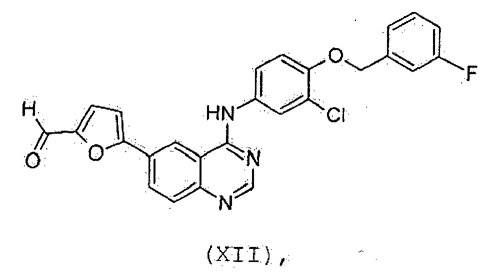
4) reacting the compound of formula (XII) with 2-(methylsulfonyl)ethanamine hydrochloride to yield Lapatinib of formula (XIII): 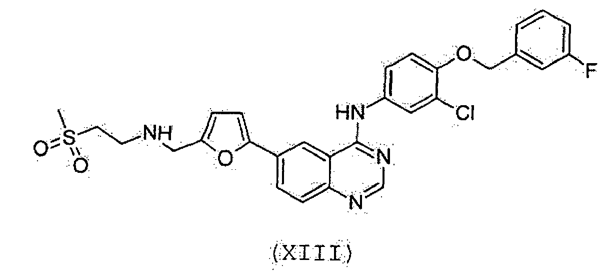
5) Optionally the Lapatinib of formula (XIII) is converted into a ditosylate salt or a monohydrate ditosylate salt.

The experimental methods (see example 8) for preparing Lapatinib and Lapatinib monohydrate ditosylate according to the method above are identical to those indicated in the examples 3-7 with the sole difference lying in the fact that it is necessary to use - in example 3 - 3-chloro-4-[(3-fluorobenzyl)oxy]aniline of formula (X) instead of 3-chloro-4-[(2-fluorobenzyl)oxy]aniline of formula (IV) and continue the synthesis with the corresponding intermediates.

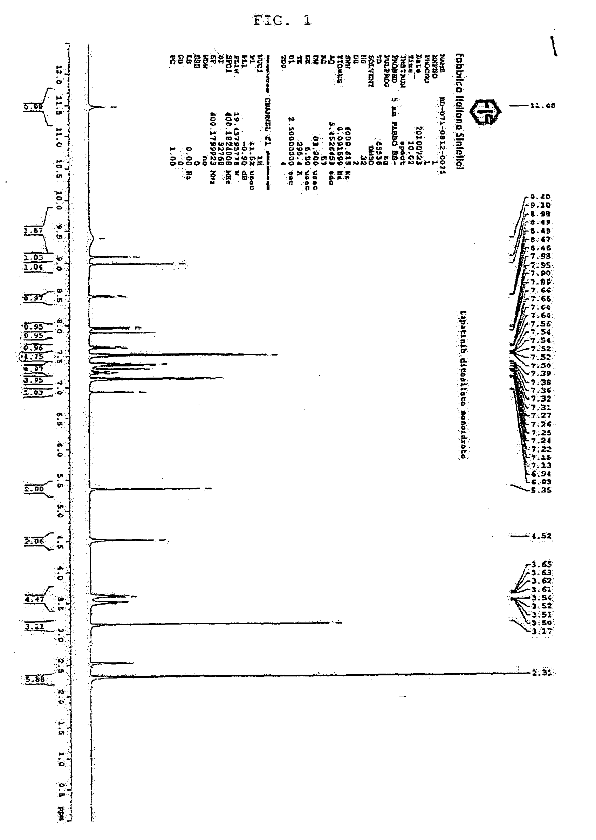

Lapatinib monohydrate ditosylate whose 1H-NMR spectrum is indicated in Figure 1 is obtained.

Thus, the use of 3-chloro-4-[(3-fluorobenzyl)oxy]aniline of formula (X) : 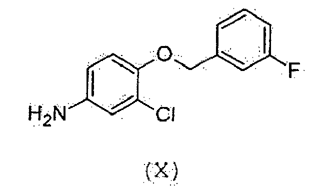 comprising less than 0.15 percent area by means of chromatographic analysis or lower than about 0.15 percent weight on weight of 3-chloro-4-[(2-fluorobenzyl)oxy]aniline of formula (IV): 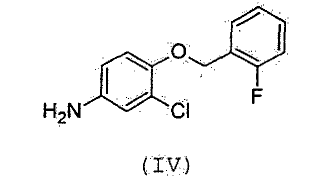 is thus essential for preparing Lapatinib or a salt thereof with the aforementioned amounts of compound (I) or a salt thereof.

Lapatinib or a salt thereof comprising less than about 0.05 percent area by means of chromatographic analysis or lower than about 0.05 percent weight on weight of compound of formula (I) or a salt thereof, obtainable through this method or other possible methods, can be used for preparing pharmaceutical compositions comprising one or more pharmacologically acceptable carriers and used in the treatment of advanced metastatic lung cancer.

### EXPERIMENTAL PART

### Example 1 - Preparation of 2-Chloro-[(2-fluorobenzyl)oxy]-4-nitrobenzene of formula (V).

### Synthesis scheme:

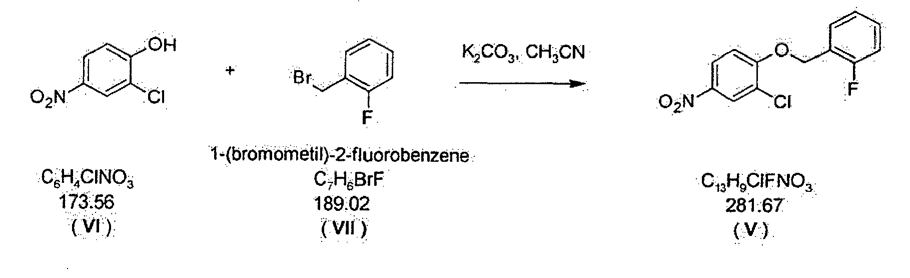

### 1-(bromomethyl)-2-fluorobenzene

In a glass flask provided with a condenser, thermometer, mechanical stirrer and nitrogen inlet, - under nitrogen flow - 22.9 g of 2-Chloro-4-nitrophenol of formula (VI), 25.0 g of 1-(bromomethyl)-2-fluorobenzene of formula (VII) and 230 mL of Acetonitrile are introduced. Stirring is carried out at 20-25 °C and 20.1 g of Potassium carbonate are added. The mixture is stirred at 60 °C for 2 hours. Upon completing the reaction cooling is carried out and the reaction mixture is poured into 230 mL of purified water pre-cooled at 0-5 °C. The precipitation of a yellowish solid is observed. The formed solid is filtered thoroughly draining the mother liquors and washing with 2x46 mL of water/acetonitrile 1:1 mixture pre-cooled at 0-5 °C. Washing is then carried out with 46 mL of n-Hexane pre-cooled at 0-5 °C. The product is dried in an oven under vacuum for 4-5 hours at 45 °C. 33.9 g of product as an almost white solid equivalent to a 91.2% yield are obtained.

### Example 2 - Preparation of 3-chloro-4-[(2-fluorobenzyl) oxy]aniline of formula (IV).

### Synthesis scheme:

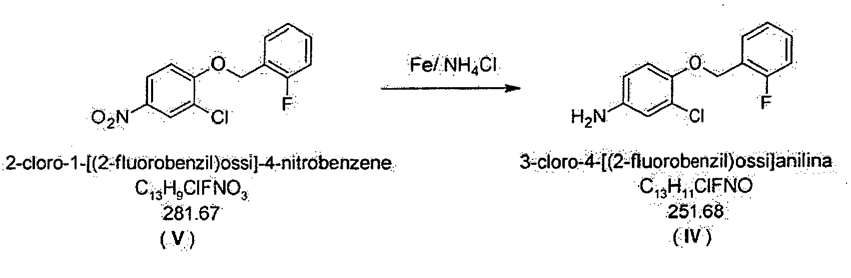

### 2-Chloro-[(2-fluorobenzyl)oxy]-4-nitrobenzene

### 3-chloro-4-[(2-fluorobenzyl)oxy]aniline

In a glass flask provided with a condenser, thermometer, mechanical stirrer and nitrogen inlet, - under nitrogen flow - 25.0 g of 2-Chloro-[(2-fluorobenzyl)oxy]-4-nitrobenzene of formula (V), 14.9 g of powder iron (MW: 55.85, 3.0 mol. equip.), 42.7 g of ammonium chloride (MW: 53.49, 9.0 mol. equiv.), 350 mL of Ethanol denatured with Methanol and 85 mL of purified water are introduced. The reaction mixture is stirred at reflux temperature (68°C) for 2 hours.

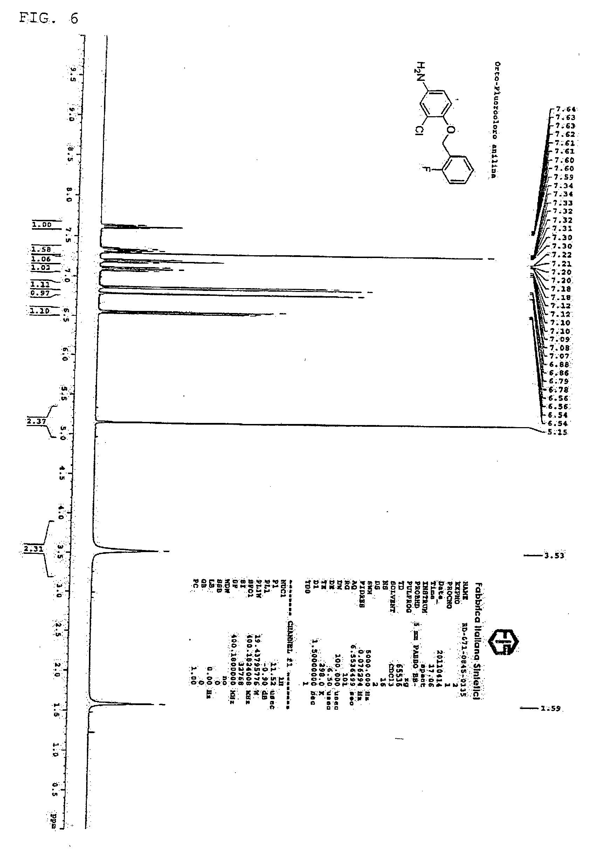

Upon completing the reaction, cooling is carried out at 20-25°C. The insoluble iron oxides are filtered on dicalite and the filtrate is evaporated to residue at an external temperature of 40-45°C. 275 mL of dichloromethane are added. The suspension is filtered on paper to remove the inorganic salts. Possible water residue is separated. The organic phase is anhydrified on anhydrous sodium sulphate, filtered and concentrated to residue eliminating the solvent thoroughly. 19.1 g of product are obtained as a pale yellow solid equivalent to an 85.5% molar yield. 1H-NMR spectrum according to figure 6.

Example 3 - Preparation of N-{3-chloro-4-[(2-fluorobenzyl)oxy]phenyl}-6-iodoquinazoline-4-amine hydrochloride of formula (III).

### Synthesis scheme:

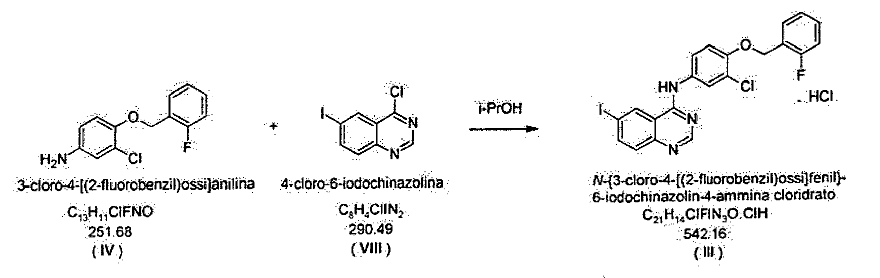

### 3-chloro-4-[(2-fluorobenzyl)oxy]aniline

### 4-chloro-6-iodoquinazoline

### N-{3-chloro-4-[(2-fluorobenzyl)oxy]phenyl}-6-iodoquinazoline-4-amine hydrochloride

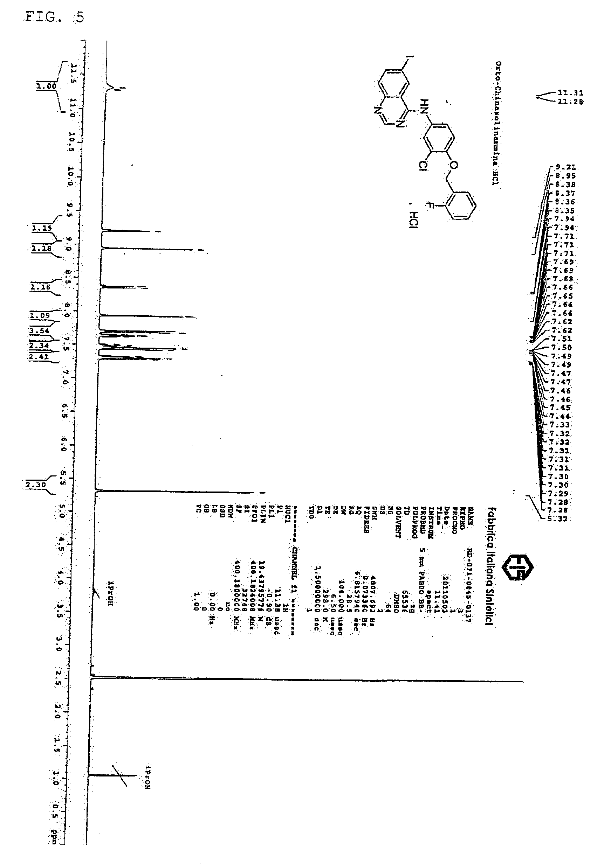

In a glass flask provided with a condenser, thermometer, mechanical stirrer and nitrogen inlet, - under nitrogen flow - 15.0 g of 3-chloro-4-[(2-fluorobenzyl) oxy]aniline of formula (IV), 17.49 g of 4-chloro-6-iodoquinazoline of formula (VIII)(available in the market), 300 mL of Isopropanol are introduced. The reaction mixture is stirred at 70 °C for 2 hrs. Upon completing the reaction cooling is carried out at ambient temperature. The formed yellow solid is filtered washing the solid with 20 mL of cold Isopropanol. 31.9 g of product equivalent to a 98.7% molar yield are obtained. 1H-NMR spectrum according to figure 5.

### Example 4 - Preparation of 5-[4-({3-chloro-4-[(2-fluorobenzyl)oxy]phenyl}amino)quinazoline-6-yl]furan-2-carbaldehyde of formula (II).

### Synthesis scheme:

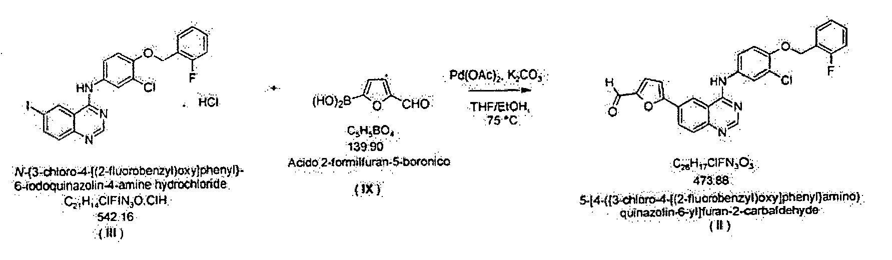

### N-{3-chloro-4-[(2-fluorobenzyl)oxy]phenyl}-6-iodoquinazoline-4-amine hydrochloride

### 2-formyl furan-5-boronic acid

### 5-[4-({3-chloro-4-[(2-fluorobenzyl)oxy]phenyl}amino)quinazoline-6-yl]furan-2-carbaldehyde

In a glass flask provided with a condenser, thermometer, mechanical stirrer and nitrogen inlet, - under nitrogen flow - 25.0 g of N-{3-chloro-4-[(2-fluorobenzyl)oxy]phenyl}-6-iodoquinazoline-4-amine hydrochloride of formula (III), 7.26 g of 2-formyl furan-5-boronic acid of formula (IX) (1.126 mol. equiv.), 0.31 g of Palladium acetate, 19.4 g of Potassium carbonate, 190 mL of absolute ethanol and 190 mL of THF are introduced.

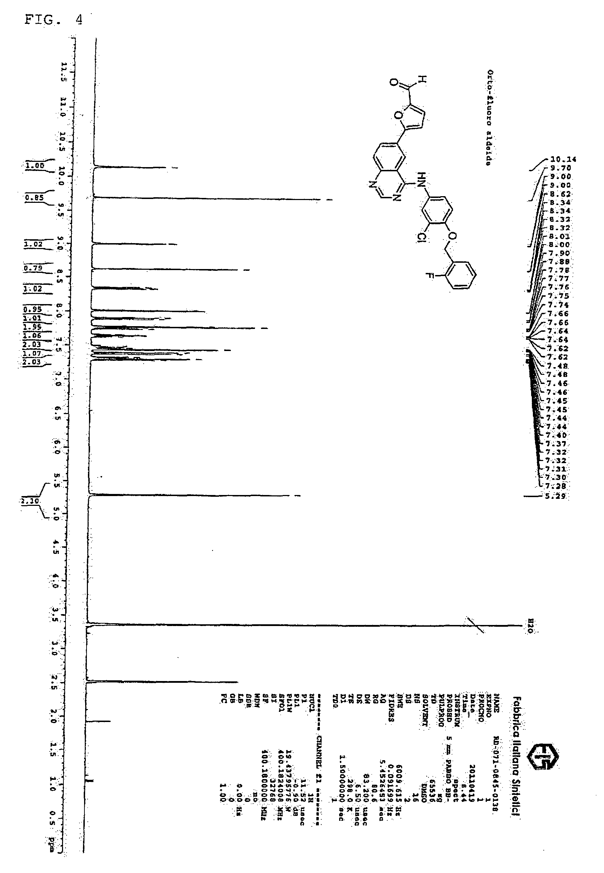

The reaction mixture is heated at T = 75 °C for 1 hr. Upon completing the reaction cooling is carried out at 50-60°C and 190 mL of Absolute ethanol and 190 mL of THF are added. Stirring is carried out at 50-60 °C for 1 hr then filtration is carried out at this temperature washing with 25 mL of Absolute ethanol and 25 mL of THF pre-heated at 50-60°C. 750 mL of purified water are added to the filtrate in one hour. Stirring is carried out at ambient temperature for 1.5 hours then Filtration is carried out washing with 25 mL of Absolute ethanol. The product is dried under vacuum at 50 °C for 7-8 hours. 21.6 g of product equivalent to a 98.8% molar yield are obtained. 1H-NMR spectrum according to figure 4.

### Example 5 - Preparation of N-{3-chloro-4-[(2-fluorobenzyl)oxy]phenyl}-6-[5-({[2-(methylsulfonyl)ethyl]amino}methyl)furan-2-yl] quinazoline-4-amine of formula (I)

### Synthesis scheme:

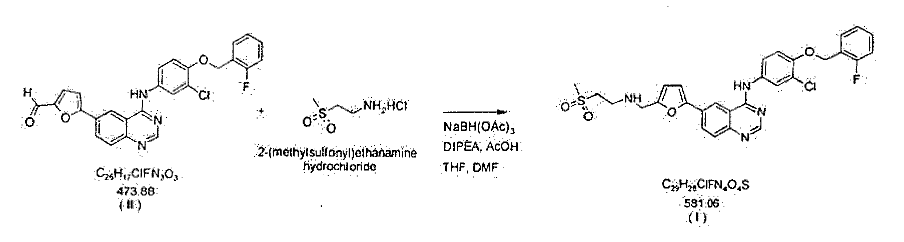

### 2-(methylsulfonyl)ethanamine hydrochloride

In a 4-neck glass flask provided with mechanical stirrer, condenser and thermometer, - under nitrogen - 15.0 g of 5-[4-(13-chloro-4-[(2-fluorobenzyl)oxy]phenyl}amino)quinazoline-6-yl]furan-2-carbaldehyde of formula (II), 8.18 g of 2-(methylsulfonyl)ethanamine hydrochloride (1.64 mol. equip.), 75 mL of THF, 15 mL of DMF, 7.22 mL of Glacial acetic acid (4 mol. equiv.) and 22.05 mL of Diisopropylamine (DIPEA) (4 mol. equiv.) were introduced. Stirring is carried out at 35°C for 1 hour and the cooling is carried out at 20-25°C and 16.77 g of Sodium triacetoxyborohydride(2.5 sol. equiv.) are added. Stirring is carried out at 25°C for 2 hours. Upon completing the reaction 225 mL of ethyl acetate are added and 45 mL of purified water are dripped in 30 minutes under stirring. 15.0 mL of 30% NaOH solution (w/w) are subsequently dripped in 30 minutes up to a pH of about 11-11.5. The phases are separated and the organic phase is washed with 2x75 mL of 25% w/w solution of Ammonium chloride and 2x45 mL of purified water. The organic phase is concentrated under vacuum up to residue.

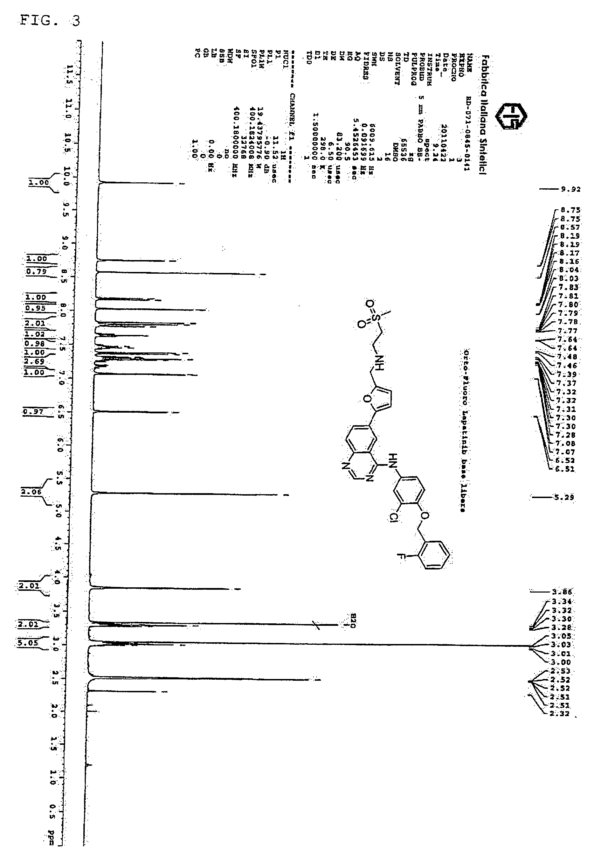

A small amount of the obtained solid equivalent to 1.0 g is taken and triturated for 1 hour in 5 mL of ethyl acetate. Filtration is carried out and the solid is washed using 2 mL of ethyl acetate and dried. 0.7 g of product are thus obtained. 1H-NMR spectrum according to figure 3.

### Example 6 - Preparation of N-{3-chloro-4-[(2-fluorobenzyl)oxy]phenyl}-6-[5-(5-{[2-(methylsulfonyl)ethyl] amino}methyl)furan-2-yl] quinazoline-4-amine ditosylate salt of formula (I).

### Synthesis scheme:

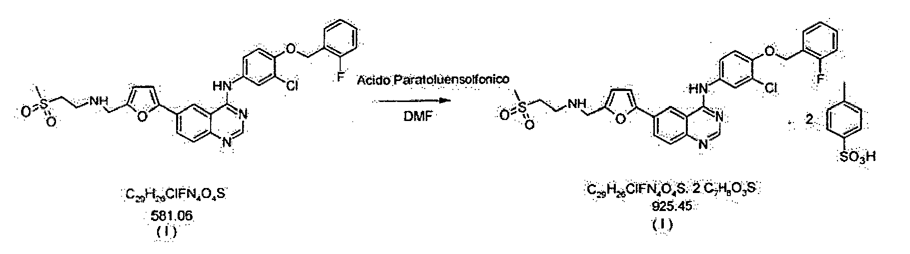

### Paratoluenesulfonic acid

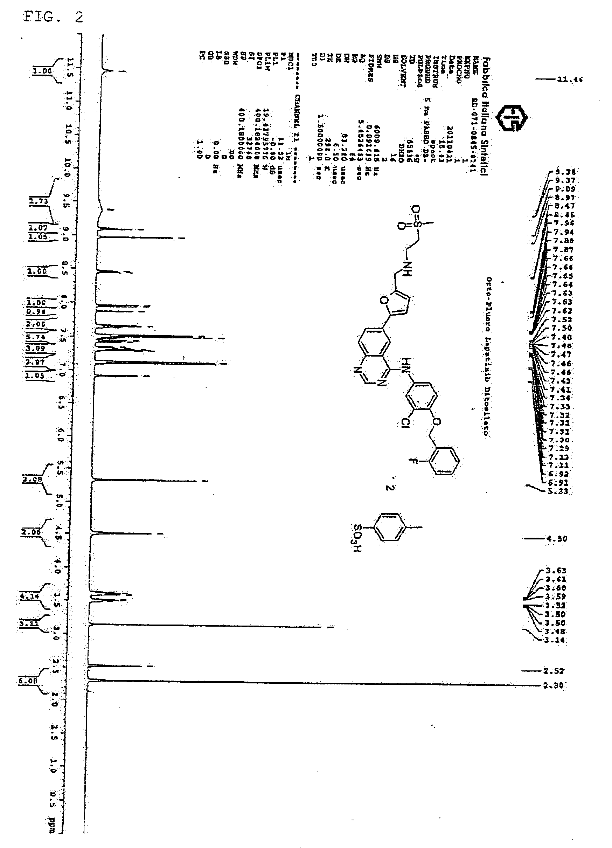

The residue obtained from the concentration of the organic phase in Example 5 constituted by the compound of formula (I) (as a free base) is recovered using 52.5 mL of dimethylformamide. Heating is carried out at 40°C for 15 minutes and filtration is carried out on a dicalite panel. The panel is washed using 39.5 ml of dimethylformamide pre-heated at 50°C. The organic phases are combined, they are brought to 40°C and 12.64 g of monohydrate Paratoluenesolfonic acid (2.1 mol. equiv.)are added to subsequent portions. Stirring is carried out at 40°C for 1, hour and then cooling is carried out in 3-4 hours at 0°C. Stirring is carried out for 1 hour a 0°C, then cooling is carried out at -10°C and then stirring is carried out for 1 hour. The suspension is filtered and the solid is washed using 9 mL of Dimethylformamide pre-cooled at -10°C. The solid is recovered using 75 mL of DMF and pulping is carried out at 40°C for 2 hours. Cooling is carried out slowly at -10°C and stirring is carried out at this temperature for 1-2 hours, the solid is filtered and washed using 9 ml of DMF pre-cooled at -10°C. The solid is dried in an oven under vacuum at 70°C for at least 10 hours. 21.4 g of product equivalent to a 73.0% molar yield are obtained. 1H-NMR spectrum according to figure 2.

### Example 7 - Preparation of N-{3-chloro-4-[(2-fluorobenzyl)oxy]phenyl}-6-[5-({[2-(methylsulfonyl)ethyl] amino}methyl)furan-2-yl] quinazoline-4-amine monohydrate ditosylate salt of formula (I - bis).

### Synthesis scheme:

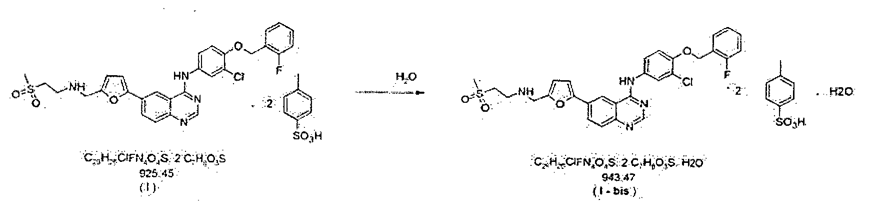

In a 4-neck glass flask provided with mechanical stirrer, condenser and thermometer, - under nitrogen - 50.0 g of N-{3-chloro-4-[(2-fluorobenzyl)oxy]phenyl}-6-[5-({[2-(methylsulfonyl)ethyl]amino}methyl)furan-2-yl] quinazoline-4-amine of formula (I) ditosylate salt and 500 mL of water were introduced. Stirring is carried out for 36 hours at ambient temperature. Filtration is carried out thoroughly draining the product and the product is washed using the mother liquors. The product is dried at ambient temperature - under nitrogen flow - in a flask provided with a stirrer.

The product is thus dried for 24 hours at 55°C up to K.F. around 1.94%. 50.5 g of product are obtained for a quantitative molar yield.

### Example 8 - Preparation of Lapatinib monohydrate ditosylate salt of formula (XIII - bis).

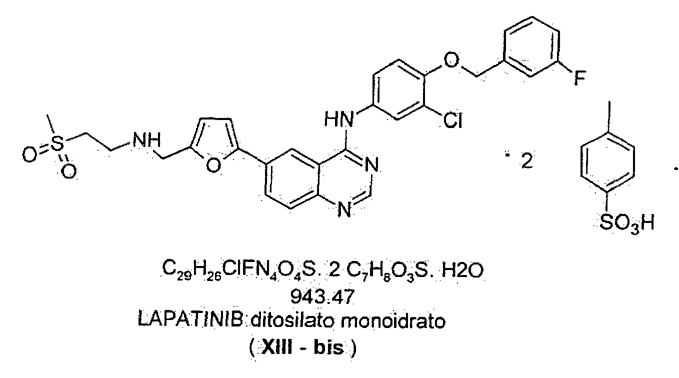

### Lapatinib monohydrate ditosylate

The preparation of Lapatinib and Lapatinib monohydrate ditosylate is performed repeating the procedures of examples 3-7 with the sole difference lying in the fact that in example 3 3-chloro-4-[(3-fluorobenzyl)oxy]aniline of formula (X) is used instead of 3-chloro-4-[(2-fluorobenzyl)oxy]aniline of formula (IV).

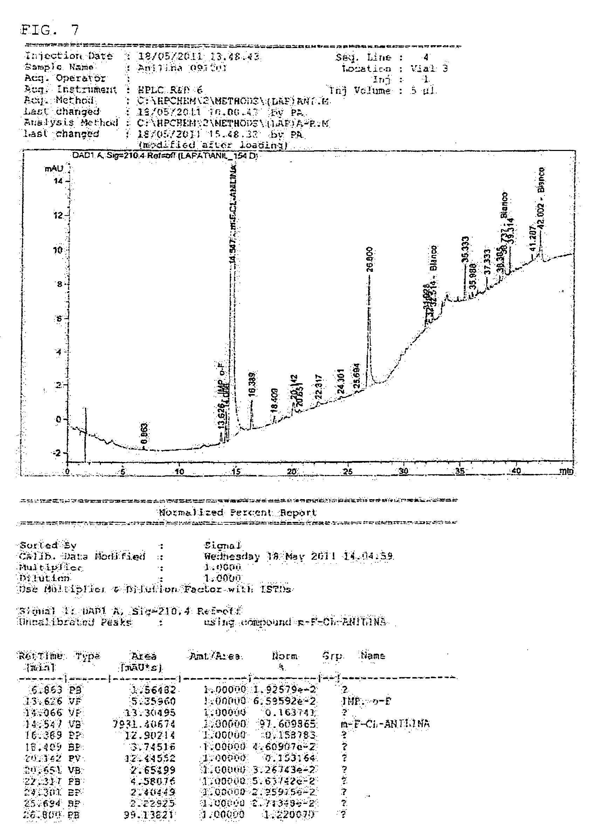

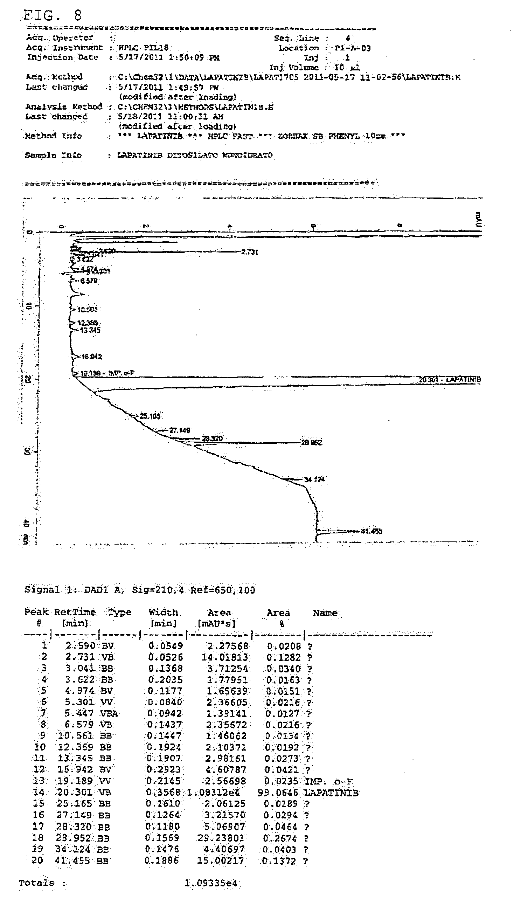

In particular starting from a purchase batch of 3-chloro-4-[(3-fluorobenzyl)oxy]aniline of formula (X) containing 0.066 percent area (HPLC) of 3-chloro-4-[(2-fluorobenzyl)oxy]aniline impurity of formula (IV) (Figure 7) Lapatinib monohydrate ditosylate of formula (XIII - bis) containing 0.024 percent area (HPLC) of impurity of formula (I -bis) is obtained through said method (Figure 8).

Figure 1 shows the 1H-NMR spectrum of the obtained Lapatinib monohydrate ditosylate whose interpretation is indicated below.

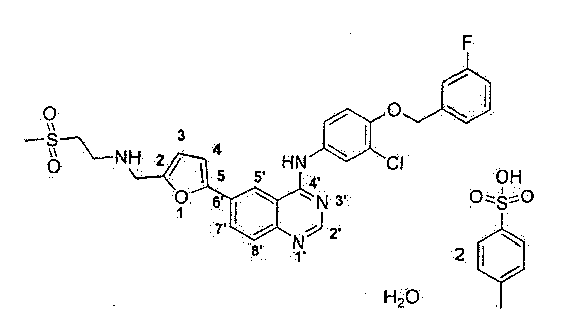

1H NMR (400 MHz, DMSO-d6) - δ 2.31 (s, 6 H, CH3 (TsOH)) ; 3.17 (s, 3 H, CH3SO2); 3.50-3.65 (m, 4 H, -SO2CH2CH2NH-); 4.52 (s, 2, NH-CH2-furan); 5.35 (s, 2 H, ArO-CH2-Ar); 6.93 (d, J = 3.4 Hz, 1 H, CH(furan)); 7.14 (d, J = 7.8 Hz, 4H, CH(TsOH)); 7.24 (dt, J = 8.8, 2.1 Hz, 1 H, Ar); 7.32 (d, J = 3.4 Hz, 1 H, CH(furan)); 7.53 (d, J = 8.0 Hz, 4 H, CH(TsOH)); 7.65 (dd, J = 8.9, 2.5 Hz, Ar); 7.90 (d, J = 2. 6 Hz, 1H, H-5'); 7.97 (d, J = 8.8 Hz, 1 H, H-8'); 8.48 (dd, J = 8.8, 1.5 Hz, 1 H, H-7'); 8.99 (s, 1 H, H-2'); 9.10 (s, 1 H, Ar); 9.40 (br. s, 1 H, NH) ; 11.48 (s, 1 H, NH).

### Example 9 - Analytic method for determining the amount of 3-chloro-4-[(2-fluorobenzyl)oxy]aniline impurity of formula (IV) in the 3-chloro-4-[(3-fluorobenzyl)oxy]aniline of formula (X):

Chromatography conditions:

| | |
|---|---|
| Column: | Waters Symmetry Shield 150x4.6 mm, 3.5 micron |
| Column temp: | 25°C |
| Mobile phase A: | H3PO4 0.1% |
| Mobile phase B: | Acetonitrile |
| Flow: | 1.2 mL/min. |

### Gradient:

| (minutes) | % A | % B |
|---|---|---|
| 0 | 85 | 15 |
| 15 | 70 | 30 |
| 20 | 66 | 34 |
| 26 | 60 | 40 |
| 40 | 0 | 100 |
| 44 | 0 | 100 |
| 45 | 85 | 15 |

| | |
|---|---|
| Detector: | UV at 210 nm |
| Post run: | 6 minutes |
| Injection volume: | 5 microL |
| Diluent: | H2O/ACN (1/1) |

Applying the conditions described above the expected retention times are as indicated below (Figure 7) :

| Compound | RT | RRT |
|---|---|---|
| | (min) | |
| impurity (IV) | 13.6 | 0.94 |
| Compound (X) | 14.5 | 1.00 |

The amount of impurity of formula (IV) is determined in Percent area.

### Example 10 - Analytic method for determining the amount of impurity of formula (I - bis) in the Lapatinib monohydrate ditosylate of formula (XIII - bis):

### Chromatography conditions:

### Analytic method obtained from WO 2010/017387 (TEVA)

| | |
|---|---|
| Column: | Zorbax SB Phenyl 100x4.6 mm, 1.8 micron or equivalent |
| Column temp: | 20°C |
| Mobile phase A: | 80% (20mM KH2PO4 pH 5.0) : 20% Acetonitrile |
| Mobile phase B: | Acetonitrile |
| Flow: | 1.5 mL/min. |
| Detector: | UV at 210 nm |
| Post run: | 10 minutes |
| Injection volume: | 5 microL |
| Diluent: | H2O/ACN (1/1) |

### Gradient:

| (minutes) | % A | % B |
|---|---|---|
| 0 | 70 | 30 |
| 20 | 70 | 30 |
| 34 | 35 | 65 |
| 42 | 35 | 65 |

Applying the conditions described above the expected retention times are as indicated below (Figure 8) :

| Compound | RT | RRT |
|---|---|---|
| | (min) | |
| o-F-Lapatinib | 19.2 | 0.95 |
| (LAP) Lapatinib | 20.3 | 1.00 |

The amount of impurity of formula (I - bis) is determined in Percent area.

It is observed that the use of the compound of formula (I) subject of the present invention in the identification and quantification of the content thereof in Lapatinib allows obtaining the product with a level of impurity of formula (I) capable of improving the quality of the drug.

## Claims

1. An N-{3-chloro-4-[(2-fluorobenzyl)oxy]phenyl}-6-[5-({[2-(methylsulfonyl)ethyl]amino}methyl)furan-2-yl] quinazoline-4-amine compound of formula (I) or a salt thereof:

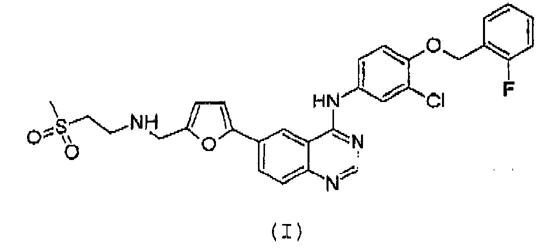

2. The compound according to claim 1 as a ditosylate salt or as a monohydrate ditosylate salt of formule (I - bis):

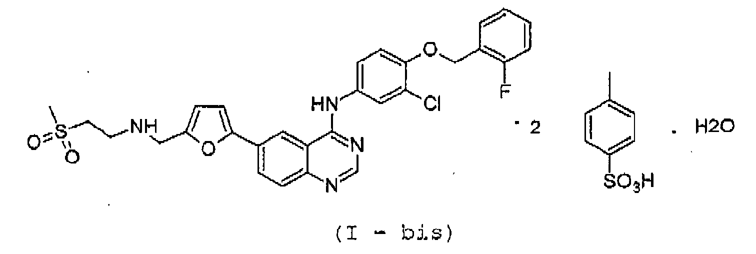

3. The compound according to claim 1 or 2 comprising from 0.01 to 5.0 percent area (HPLC) of Lapatinib or a salt thereof.

4. The compound according to any one of claims 1 to 3 having at least a 95.0 HPLC purity (area percent).

5. A method for preparing the compound of formula (I) according to any one of claims 1 to 4 comprising the hollowing steps:
A) reacting 3-chloxo-9-[(2-fluorobenzyl)oxy]aniline of formula (IV): 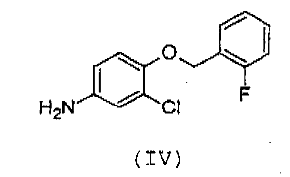 with 4-chloro-6-iodoquinazoline of formula (VIII): 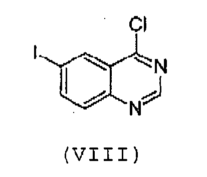 to yield N-{3-chloro-4-[(2-fluorobenzyl)oxy]phenyl}-6-iodoquinazoline-4-amine hydrochloride of formula (III): 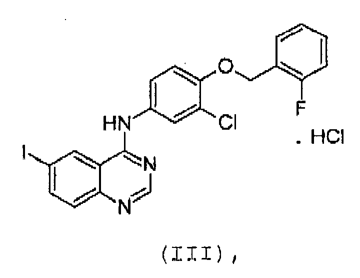
B) reacting the compound of formula (III) with the 2-formyl furan-5-boronic acid of formula (IX): 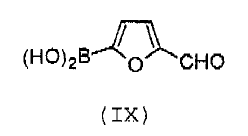 to yield 5-[4-({3-chloro-4-[(2-fluorobenzyl)oxy]phenyl}amino)quinazoline-6-yl]furan-2-carbaldehyde of formula (II): 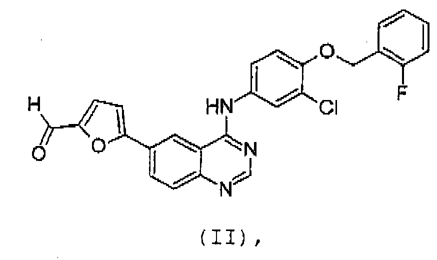
C) reacting the compound of formula (II) with 2-(methylsulfonyl)ethanamine hydrochloride to yield the compound of formula (I);
D) optionally, conversion of the compound of formula (I) into a ditosylate salt or a monohydrate ditosylate salt.

6. Method according to claim 5 wherein in step (b) the filtration of the inorganic salts present in the reaction mixture is carried out at a temperature comprised between 50°C and 60°C.

7. The compound N-{3-chloro-4-[(2-fluorobenzyl)oxy]phenyl}-6-iodoquinazoline-4-amine hydrochloride of formula (III): 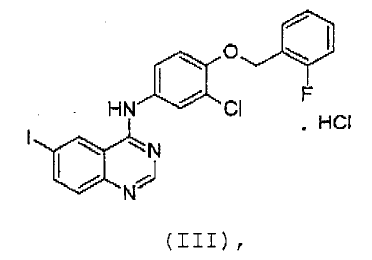 or in a free base form; 5-[4-({3-chloro-4-[(2-fluorobenzyl)oxy]phenyl} amino)quinazoline-6-yl]furan-2-carbaldehyde compound of formula (II) and salts thereof:

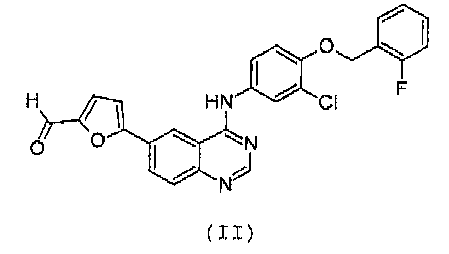

8. A method for detecting the compound of formula (I) according to claim 1 in Lapatinib or a salt thereof comprising:
a) adding a known amount of compound of formula (I) or a salt thereof to the Lapatinib sample or a salt thereof,
b) carrying out HPLC analysis of the Lapatinib sample or a salt thereof of step a),
c) detecting the HPLC peak of the compound of formula (I);
or,
a1) analysing the compound of formula (I) or a salt thereof by means of HPLC,
b1) analysing the Lapatinib sample or a salt thereof by means of HPLC,
c1) detecting the HPLC peak of the compound of formula (I) by comparing the retention times or relative retention times.

9. Method for the quantification of the compound of formula (I) or salt thereof according to claim 1 in Lapatinib or a salt thereof comprising:
i) measuring the peak area corresponding to the compound of formula (I) in a Lapatinib sample or a salt thereof having an unknown amount of this compound by means of HPLC:
ii) measuring the peak area corresponding to a "reference standard" containing a known amount of compound of formula (I) or a salt thereof by means of HPLC;
iii) defining the amount of compound of formula (I) in Lapatinib or a salt thereof comparing the area measured in step a) with that measured in step b).

10. Use of the compound of formula (I) according to any one of claims 1 to 4 as a "reference marker" or "reference standard" for the identification and/or the quantification of the same compound of formula (I) in Lapatinib or a salt thereof.

## Patentansprüche

1. N-{3-Chlor-4-[(2-fluorbenzyl)oxy]phenyl}-6-[5-({[2-(methylsulfonyl)ethyl]amino}methyl)furan-2-yl]quinazolin-4-amin-Verbindung der Formel (I) oder ein Salz davon:

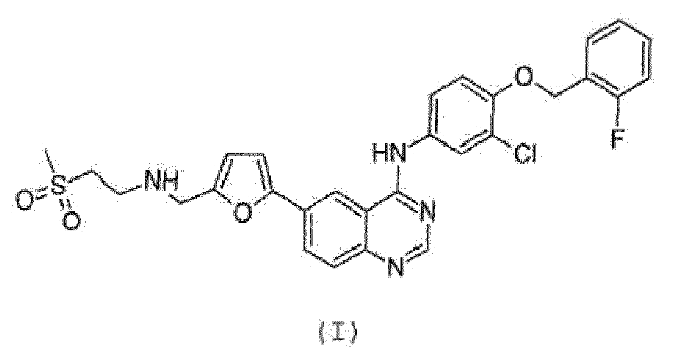

2. Verbindung nach Anspruch 1 als ein Ditosylatsalz oder als ein Monohydrat Ditosylatsalz der Formel (I - bis):

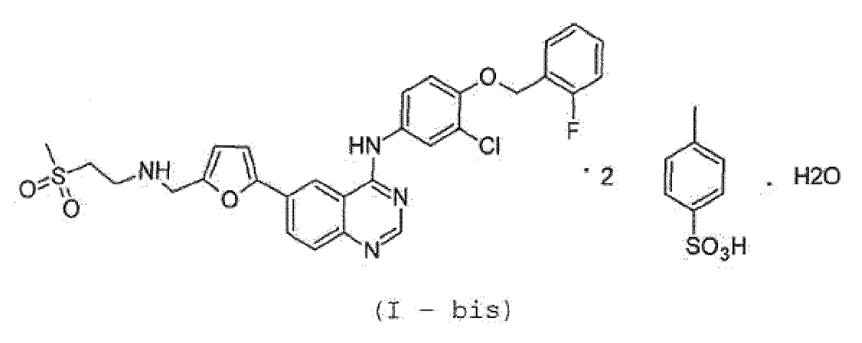

3. Verbindung nach Anspruch 1 oder 2 umfassend von 0,01 bis 5,0 Prozent Fläche (HPLC) von Lapatinib oder einem Salz davon.

4. Verbindung nach einem der Ansprüche 1 bis 3, welche mindestens eine HPLC Reinheit von 95,0 (Flächenprozent) aufweist.

5. Verfahren zur Zubereitung der Verbindung der Formel (I) nach einem der Ansprüche 1 bis 4 umfassend die folgenden Schritte:
A) Reagieren von 3-Chlor-4-[(2-Fluorbenzyl)oxy]anilin der Formel (IV): 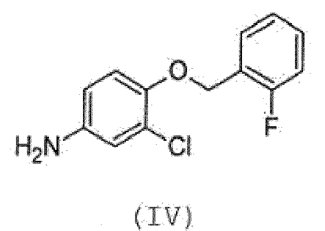 mit 4-Chlor-6-iodquinazolin der Formel (VIII): 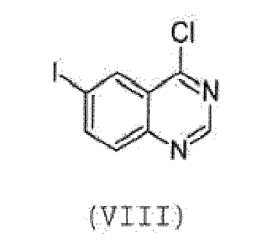 um N-{3-Chlor-4-[(2-fluorbenzyl)oxy]phenyl}-6-iodquinazolin-4-amin Hydrochlorid der Formel (III) zu erhalten: 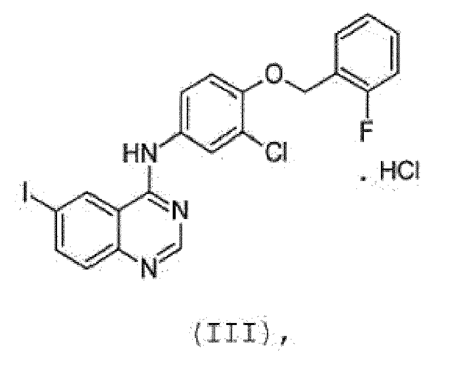
B) Reagieren der Verbindung der Formel (III) mit der 2-Formyl Furan-5-Boronsäure der Formel (IX): 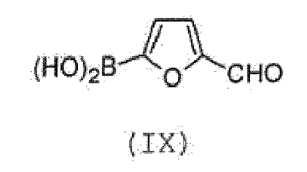 um 5-[4({3-Chlor-4-[(2-Fluorbenzyl)oxy]phenyl}amino)quinazolin-6-yl]furan-2-carbaldehyd der Formel (II) zu erhalten: 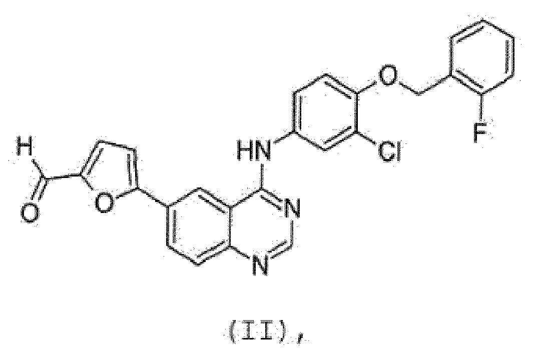
C) Reagieren der Verbindung der Formel (II) mit 2-(Methylsulfonyl)ethanamin Hydrochlorid um die Verbindung der Formel (I) zu erhalten;
D) gegebenenfalls, Umwandlung der Verbindung der Formel (I) in ein Ditosylatsalz oder ein Monohydrat Ditosylatsalz.

6. Verfahren nach Anspruch 5, wobei in Schritt (b) die Filtration der anorganischen Salze, welche in dem Reaktionsgemisch vorhanden sind, bei einer Temperatur zwischen 50°C und 60°C durchgeführt wird.

7. Verbindung N-{3-Chlor-4-[(2-fluorbenzyl)oxy]phenyl}-6-iodquinazolin-4-amin Hydrochlorid der Formel (III): 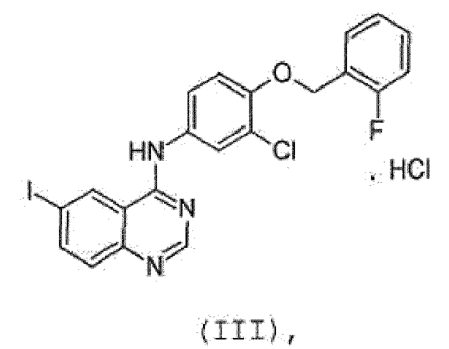 oder in einer freien Basenform;
5-[4({3-Chlor-4-[(2-Fluorbenzyl)oxy]phenyl}amino)quinazolin-6-yl]furan-2-carbaldehyd Verbindung der Formel (II) und Salze davon:

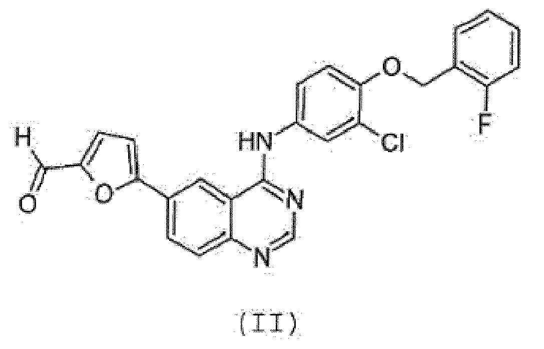

8. Verfahren zum Detektieren der Verbindung der Formel (I) nach Anspruch 1 in Lapatinib oder einem Salz davon umfassend:
a) Zugeben einer bekannten Menge der Verbindung der Formel (I) oder eines Salzes davon zu der Lapatinib-Probe oder einem Salz davon,
b) Durchführen einer HPLC-Analyse von der Lapatinib-Probe oder einem Salz davon von Schritt a),
c) Detektieren des HPLC-Peaks der Verbindung der Formel (I);
oder
a1) Analysieren der Verbindung der Formel (I) oder eines Salzes davon mittels HPLC,
b1) Analysieren der Lapatinib-Probe oder des Salzes davon mittels HPLC,
c1) Detektieren des HPLC-Peaks der Verbindung der Formel (I) durch Vergleichen der Retentionszeiten oder relativen Retentionszeiten.

9. Verfahren zur Quantifizierung der Verbindung der Formel (I) oder eines Salzes davon nach Anspruch 1 in Lapatinib oder einem Salz davon umfassend:
i) Messen der Peak-Fläche, welche der Verbindung der Formel (I) entspricht, in einer Lapatinib-Probe oder einem Salz davon, welche(s) eine unbekannte Menge dieser Verbindung enthält, mittels HPLC;
ii) Messen der Peak-Fläche, welche einem "Referenz-Standard" entspricht, welcher eine bekannte Menge der Verbindung der Formel (I) oder eines Salzes davon enthält, mittels HPLC;
iii) Definieren der Menge der Verbindung der Formel (I) in Lapatinib oder einem Salz davon durch Vergleichen der in Schritt a) gemessenen Fläche mit der, die in Schritt b) gemessen wurden.

10. Verwendung der Verbindung der Formel (I) nach einem der Ansprüche 1 bis 4 als ein "Referenz-Marker" oder "Referenz-Standard" für die Identifizierung und/oder die Quantifizierung der selben Verbindung der Formel (I) in Lapatinib oder einem Salz davon.

## Revendications

1. Composé N-{3-chloro-4-[(2-fluorobenzyl)oxy]phényl}-6-[5-({[2-(méthylsulfonyl)éthyl]amino} méthyl)furan-2-yl]quinazoline-4-amine de formule (I) ou sel de celui-ci :

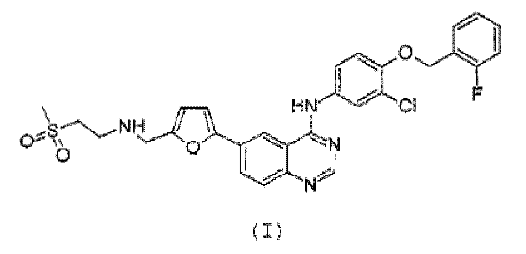

2. Composé selon la revendication 1 sous forme d'un sel ditosylate ou d'un sel ditosylate monohydraté de formule (I - bis) :

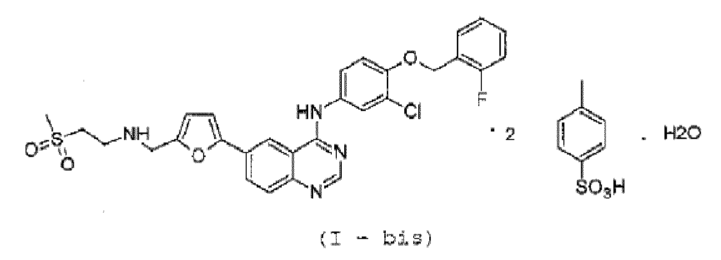

3. Composé selon la revendication 1 ou 2 comprenant de 0,01 à 5,0 pourcent en aire (CLHP) de Lapatinib ou d'un sel de celui-ci.

4. Composé selon l'une quelconque des revendications 1 à 3 ayant au moins une pureté CLHP de 95,0 (pourcent en aire).

5. Procédé pour préparer le composé de formule (I) selon l'une quelconque des revendications 1 à 4 comprenant les étapes suivantes :
A) réaction de la 3-chloro-4-[(2-fluorobenzyl)oxy]aniline de formule (IV) : 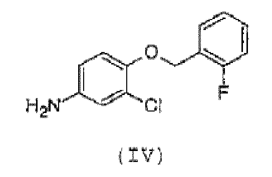 avec la 4-chloro-6-iodoquinazoline de formule (VIII) : 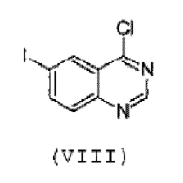 pour produire le chlorhydrate de N-{3-chloro-4-[(2-fluorobenzyl)oxy]-phényl}-6-iodoquinazoline-4-amine de formule (III) : 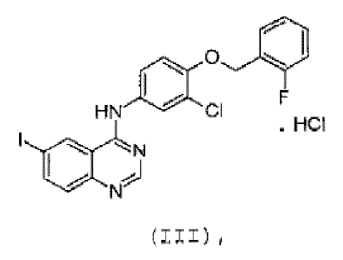
B) réaction du composé de formule (III) avec l'aide 2-formyl furane-5-boronique de formule (IX) : 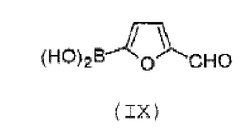 pour produire le 5-[4-({3-chloro-4-[(2-fluorobenzyl)oxy]phényl}amino)-quinazolin-6-yl]furane-2-carbaldéhyde de formule (II) : 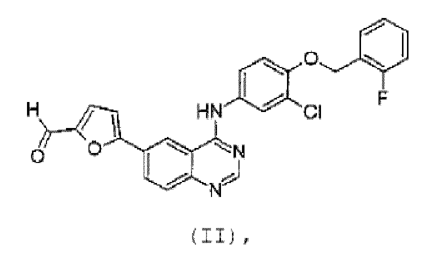
C) réaction du composé de formule (II) avec le chlorhydrate de 2-(méthylsulfonyl)éthanamine pour produire le composé de formule (I) ;
D) éventuellement, conversion du composé de formule (I) en un sel ditosylate ou un sel ditosylate monohydraté.

6. Procédé selon la revendication 5 où, dans l'étape (b), la filtration des sels inorganiques présents dans le mélange réactionnel est conduite à une température comprise entre 50°C et 60°C.

7. Composé chlorhydrate de N-{3-chloro-4-[(2-fluorobenzyl)-oxy]phényl}-6-iodoquinazoline-4-amine de formule (III) : 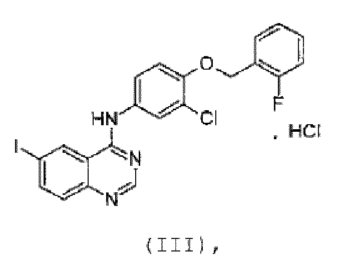 ou sous une forme de base libre ;
composé 5-[4-({3-chloro-4-[(2-fluorobenzyl)oxy]phényl}amino)-quinazolin-6-yl]furane-2-carbaldéhyde de formule (II) et ses sels:

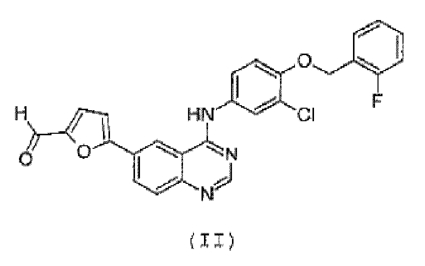

8. Procédé pour détecter le composé de formule (I) selon la revendication 1 dans le Lapatinib ou un sel de celui-ci comprenant :
a) l'addition d'une quantité connue de composé de formule (I) ou d'un sel de celui-ci à l'échantillon de Lapatinib ou un sel de celui-ci,
b) la mise en oeuvre d'une analyse par CHLP de l'échantillon de Lapatinib ou d'un sel de celui-ci de l'étape a),
c) la détection du pic en CLHP du composé de formule (I) ;
ou,
a1) l'analyse du composé de formule (I) ou d'un sel de celui-ci par CLHP,
b1) l'analyse de l'échantillon de Lapatinib ou d'un sel de celui-ci par CLHP,
c1) la détection du pic en CLHP du composé de formule (I) en comparant les temps de rétention ou les temps de rétention relatifs.

9. Procédé pour la quantification du composé de formule (I) ou d'un sel de celui-ci selon la revendication 1 dans le Lapatinib ou un sel de celui-ci comprenant :
i) la mesure de l'aire de pic correspondant au composé de formule (I) dans un échantillon de Lapatinib ou un sel de celui-ci ayant une quantité inconnue de ce composé par CLHP ;
ii) la mesure de l'aire de pic correspondant à un "étalon de référence" contenant une quantité connue de composé de formule (I) ou d'un sel de celui-ci par CLHP ;
iii) la définition de la quantité de composé de formule (I) dans le Lapatinib ou un sel de celui-ci en comparant l'aire mesurée dans l'étape a) avec celle mesurée dans l'étape b).

10. Utilisation du composé de formule (I) selon l'une quelconque des revendications 1 à 4 comme "marqueur de référence" ou "étalon de référence" pour l'identification et/ou la quantification du même composé de formule (I) dans le Lapatinib ou un sel de celui-ci.
